# EUROPEAN PATENT SPECIFICATION

(11) **EP 2 058 014 B1**
(45) Date of publication and mention of the grant of the patent: **12.08.2015**
(21) Application number: 07805775.9
(22) Date of filing: 28.08.2007
(51) Int. Cl.: A61L 27/06, A61L 27/56, A61F 2/44, A61F 2/30, A61L 27/30, A61F 2/28

(54) **COMPOSITE ARTIFICIAL BONE**
KÜNSTLICHER KNOCHENVERBUND
OS ARTIFICIEL COMPOSITE

(30) Priority: 31.08.2006 JP 2006235019
(43) Date of publication of application: 13.05.2009
(73) Proprietor: Japan Science and Technology Agency, Kawaguchi-shi, Saitama 332-0012 (JP); Chubu University Educational Foundation, Kasugai-shi, Aichi 487-8501 (JP); Advanced Medix Inc., Kyoto 617-0841 (JP)
(72) Inventor: KOKUBO, Tadashi, Kyoto 617- 0841 (JP)
(74) Representative: Hart-Davis, Jason
(86) International application number: PCT/JP2007/000920
(87) International publication number: WO 2008/026316

(56) References cited:
- JP-A- 06 007 388
- JP-A- 10 043 216
- JP-A- 63 115 557
- JP-A- 2002 285 203
- JP-A- 2003 094 109
- US-A1- 2006 002 810
- LAPTEV A ET AL: "STUDY OF PRODUCTION ROUTE FOR TITANIUM PARTS COMBINING VERY HIGH POROSITY AND COMPLEX SHAPE", POWDER METALLURGY, MANEY PUBLISHING, LONDON, GB, vol. 47, no. 1, 1 January 2004 (2004-01-01), pages 85-92, XP001208014, ISSN: 0032-5899, DOI: 10.1179/003258904225015536
- KANETAKE N.: 'Porous Kinzoku no Kogyo Riyo to Kongo no Kanosei' SOKEIZAI vol. 47, no. 6, June 2006, pages 1 - 5, XP003021266

## Description

### TECHNICAL FIELD

The present invention relates to a method of producing an artificial bone made of titanium or a titanium alloy, in which a dense part and a porous part are integrated.

### BACKGROUND ART

Titanium is expected as a material for an artificial bone because it has higher mechanical strength than ceramics and resins, and is able to have a bone forming ability only through an alkaline treatment. It is desired that a material for an artificial bone is a porous body having a porosity of 50% or higher, because of the necessity of forming a living bone and bonding to a surrounding living bone after the material is implanted into a living body. The mechanical strength that is required for an artificial bone is 40 MPa or more for substitutes for spine although it depends on the site where it is implanted. It is known that a titanium porous body is obtained by sintering after mixing a titanium powder with a pore forming material as is necessary and pressure-molding the mixture (Patent document 1).

Patent document 1: JP2002-285203-A US 2006/002810 A1 discloses a method of producing a metallic artificial bone. The artificial bone is at least partially porous and exhibits a predetermined pore character. The forming of metal articles with layers of differing porosity is obtained by forming layers containing extractable particulate of varying characteristics. The layers may also be completely free of the extractable particulate to provide a dense layer.

### DISCLOSURE OF THE INVENTION

### PROBLEM TO BE SOLVED BY THE INVENTION

However, when a porous body is used alone even if it is made of a metal, chips and breakages are likely to occur in a corner or an edge line, so that it has poor reliability. In addition, since the modulus of elasticity of a living bone largely differs depending on the site, as is evident from 10 to 20 GPa for a cortical bone, and 0.2 to 0.3 GPa for a cancellous bone, it is difficult to adapt the modulus of elasticity of an artificial bone to these values.

Therefore, it is an object of the present invention to provide **a method of producing** an artificial bone which is easy to bond to a living bone and has a mechanical property approximate to that of a living bone.

### MEANS FOR SOLVING THE PROBLEM

In order to achieve the above object, the artificial bone **produced by the method** of the present invention comprises
a dense part made of titanium or a titanium alloy, in the shape of a frame that is approximate to a part of an outer face of a living bone, having a density of 95% or more, and
a porous part made of sintered particles of titanium or a titanium alloy, in the shape approximate to the remaining part of the living bone, having a porosity of 40% or more.

The dense part and the particles of the porous part are sintered to each other at an interface between the dense part and the porous part.

The titanium alloy for the porous part may have the same or different composition as that for the dense part.

The part of an outer face of a living bone is typically a part of a cortical bone, and the remaining part of the living bone is typically the remaining part of the cortical bone and a cancellous bone of the living bone.

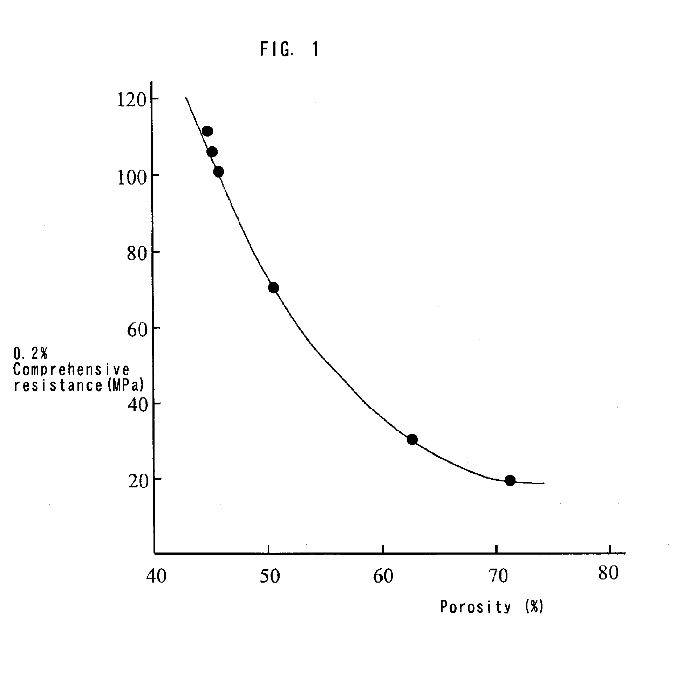

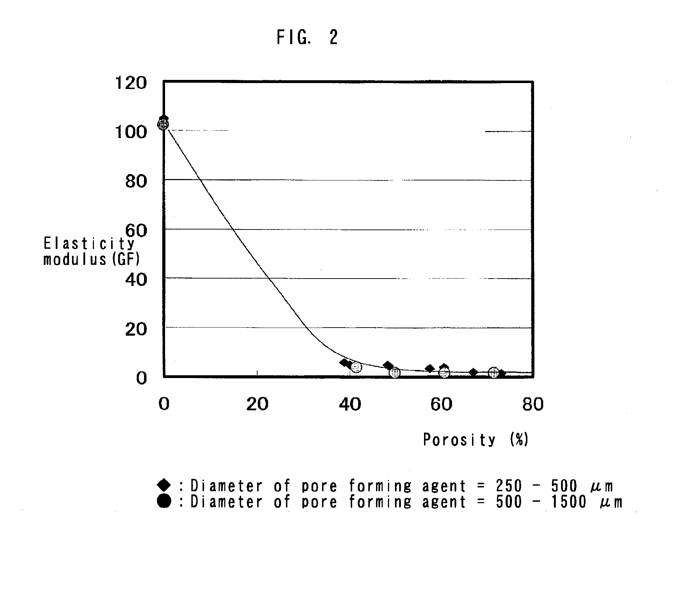

According to an experiment by the present inventor, it is necessary to make the porosity 55% or less as shown in Fig. 1, for making a pure titanium porous body have a compressive strength of 40 MPa or more. As shown in Fig. 2, the modulus of elasticity decreases with the porosity at porosities of up to 40%, while it gradually decreases at porosities exceeding 40% and reaches about 2 GPa at a porosity of 70%.

On the other hand, since the artificial bone **produced by the method** of the present invention has a dense part in the shape of a frame and a porous part having a porosity of 40% or more, biological tissues such as a living bone or a body fluid enter the porous part through an opening of the frame, and bond to the porous part. Since the dense part supports the load, the compressive strength is high for the high porosity as a whole. Also, by appropriately defining the volume ratio between the porous part and the dense part, it is possible to adapt the modulus of elasticity to that of a living bone. Further, when the part corresponding to a corner, an edge line or a surface of a living bone is composed of the dense part, chips and breakages can be prevented. Furthermore, when a sharp projection is formed on one end face of the dense part integrally, it is possible to achieve initial fixation by making this projection break into a living bone.

Such an artificial bone is produced, **in a method of the invention according to appended claim 1**, by calcining after combining a shaped body (a) of titanium or a titanium alloy powder not containing a pore forming agent (hereinafter, referred to as a "titanium powder and the like"), and a shaped body (b) of a mixture containing a titanium powder and the like and a pore forming agent.

According to this production method, since the part derived from the shaped body (a) (A) does not contain a pore forming agent, it becomes a dense part after calcining. On the other hand, since the part derived from the shaped body (b) contains a pore forming agent, it becomes a porous part after calcining. In addition, since the shaped body (a), and the shaped body (b) are combined and calcined together, particles inside each shaped body are sintered together, and at the same time, particles inside the shaped body (a) and particles inside the shaped body (b) are sintered at the interface. Therefore, the dense part and the porous part are joined without intervened by any other matter than a transition layer therebetween.

Shaping into each shaped body may be conducted before combining with the other shaped body, or may be conducted simultaneously with combining. An individual shaping method for each shaped body may be known appropriate means including injection molding, pressure molding, extrusion molding, cast molding and sheet forming. In particular, it is preferred that the shaped body (b) is combined with the shaped body (a) simultaneously with the shaping by placing the shaped body (a) in a die and charging the remaining space with the powder mixture, and then conducting pressure molding. This allows combination with the shaped body (b) even when the shape of the shaped body (a) is complicated. When combining is made after conducting molding individually, it is preferred to conduct fluid-pressure molding prior to calcining after combining.

Either one or both of the shaped body (a) and the shaped body (b) may contain an organic binder, and when both of them contain organic binders, the organic binders may be the same or different from each other. When both of them contain binders, the pressure molding is preferably conducted at a temperature at which the binders get soft. This is because particles in the interface will adhere to each other more closely at the molding stage, and facilitate progress of sintering.

### EFFECT OF THE INVENTION

Since the artificial bone **produced by the method** of the present invention is easy to bond to a living bone, and has a mechanical property that is approximate to that of a living bone, the load on a patient who wears the same is significantly reduced.

### BRIEF DESCRIPTION OF THE DRAWINGS

Fig. 1 is a graph showing a relation between the porosity and the compressive resistance of a titanium porous body.
Fig. 2 is a graph showing a relation between the porosity and the modulus of elasticity of a titanium porous body.
Fig. 3 is a drawing showing an initial step of a production method of an artificial bone according to Example 1.
Fig. 4 is a drawing showing an intermediate step of the same.
Fig. 5 is a CT image picture of a sintered body obtained in the intermediate step.
Fig. 6 (a) is a drawing showing an initial step of an artificial bone according to Example 3, and Fig. 6(b) is an enlarged view of B part in Fig. 6(a).
Fig. 7 is drawings showing a production method of an artificial bone according to **the Reference** Example, in which Fig. 7(a) is a picture of a dense part alone, Fig. 7(b) is a picture of a porous part alone, and Fig. 7(c) is a picture of an artificial bone.

### BEST MODES FOR CARRYING OUT THE INVENTION

An organic binder is composed, for example, of 0 to 50 vol% of polyacetal, 0 to 50 vol% of polypropylene and 50 to 70 vol% of waxes. The pressure at which pressure molding is conducted after placing a shaped body (a) in a die, and charging the remaining space with a powder mixture which is to be a shaped body (b) is preferably 20 to 100 MPa. When the pressure is less than 20 MPa, binding between a porous part and a dense part is insufficient, whereas when the pressure exceeds 100 MPa, the load exerted on the shaped body (a) is so large that the shaped body (a) may be broken. When shaping is conducted while the organic binder is contained, it is preferred to remove the organic binder by heating in atmospheric air before calcining the combined body. Final sintering is conducted at a temperature ranging from 1200 to 1400°C in a nonoxidative atmosphere.

### [Example 1]

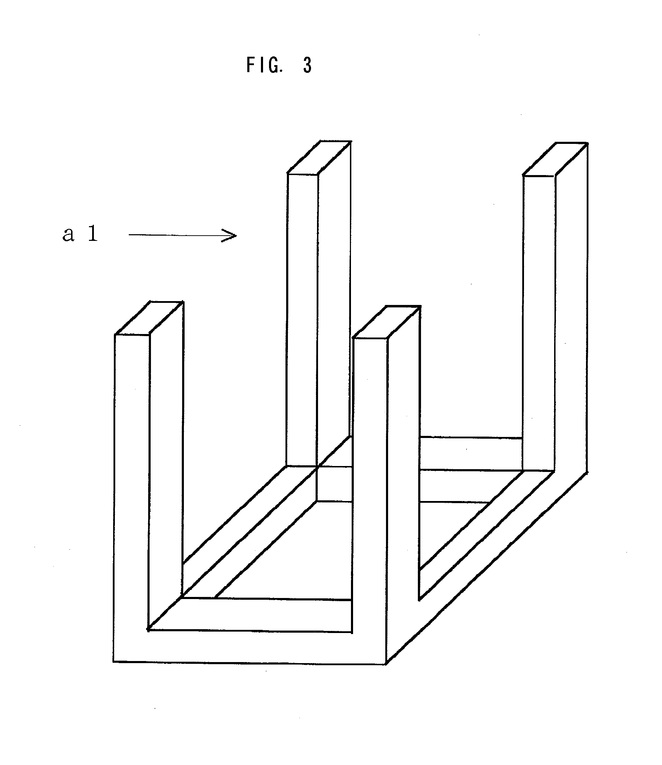

A pure titanium powder of Japanese Industrial Standard II (corresponding to US standard ASTM-G1) having a maximum particle size of 45 µm, and an organic binder composed of 20 vol% of polyacetal, 20 vol% of polypropylene and 60 vol% of waxes were mixed in a volume ratio of 65 : 35 to prepare a mixture for dense part. The volume ratio was calculated from the real density and the weight of each component. Then the mixture for dense part was subjected to injection molding at 165°C to obtain a shaped body (a1) having an outer dimension of 9.6 mm × 12.0 mm × 24.0 mm (width × depth × height) in which a square frame part formed of right quadrangular prisms of 1.50 mm wide each is provided at its four corners with standing legs of right quadrangular prisms having the same width as shown in Fig. 3.

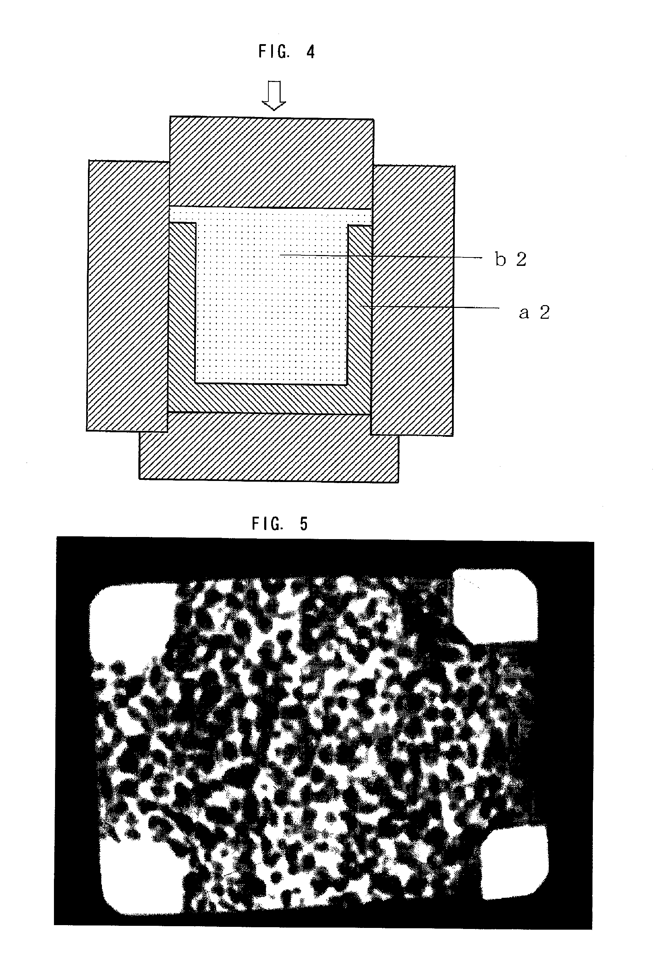

Separately, the pure titanium powder and ammonium hydrogen carbonate having a particle size adjusted to 500 to 1500 µm by means of a sieve were mixed in a volume ratio of 34 : 66, to obtain a mixture for porous part. As shown in Fig. 4, the shaped body (a1) was placed in a die having an inner dimension slightly larger than the outer dimension of the shaped body (a1), and the remaining space was charged with the mixture for porous part, and a pressure of 90 MPa was applied, to give a combined body in which a shaped body (b1) of an approximately rectangular parallelepiped was formed inside the shaped body (a1).

This combined body was calcined by retaining at 1250°C for two hours in an argon gas atmosphere. A CT image of the obtained sintered body demonstrated that the edge line part (dense part) and the inner part (porous part) were sintered while shapes originating from the shaped bodies (a1) and (b1) were respectively maintained as shown in Fig. 5. Also, particles in the interface were sintered in such a degree that boundaries thereof were difficult to be recognized, and an artificial bone of 10 mm x 8 mm x 20 mm in which the dense part and the porous part were integrated was obtained. A plurality of CT section images of this artificial bone were taken, and the maximum diameter of certain direction of pores was measured (maximum length of the gap part by a line of certain direction) on the obtained images. The pore diameter of the porous part fell within the range of 200 to 500 µm. This artificial bone was compressed in the axial direction at a compression speed of 1 mm/min. and the compressive strength was found to be 53.3 MPa. Separately, the shaped bodies (a1) and (b1) were molded individually without being combined with each other, and calcined in the same condition as described above. The density and the porosity of the obtained sintered bodies were calculated from the weight and the real density of titanium. The density of the sintered body corresponding to the dense part was 96%, and the porosity of the sintered body corresponding to the porous part was 60%. Based on values of 108 GPa and 4.2 GPa that were obtained by individually measuring the modulus of elasticity of the dense part and the porous part, the modulus of elasticity of the entire sintered body was calculated. The result was 15.8 GPa.

Next, the obtained artificial bone was immersed in a 5 M NaOH aqueous solution at 60°C for 24 hours, and sequentially immersed in pure water at 40°C for 12 hours, and then heated for an hour in an air furnace at 600°C. The artificial bone was taken out of the furnace, and immersed in a simulated body fluid having an inorganic ion concentration approximately equal to that of a human body fluid (Japanese Patent No. 2775523, column 6, lines 43 to 49), and deposition of an apatite phase was observed in the dense part and the porous part after three days.

### [Example 2]

A combined body in which a shaped body (b2) of an approximately rectangular parallelepiped was molded inside the shaped body (a1) was obtained in the same condition as that of Example 1 except that the volume ratio between the titanium powder and ammonium hydrogen carbonate in the mixture for porous part was 43 : 57. This combined body was calcined by retaining for two hours in an argon gas atmosphere at 1250°C. The density of the sintered body corresponding to the dense part was 96%, and the porosity of the sintered body corresponding to the porous part was 50.5%.

The obtained artificial bone was subjected to an alkali and heat treatment, and immersed in a simulated body fluid in a similar manner as in Example 1, and deposition of an apatite phase was observed in the dense part and in the porous part after three days.

### [Example 3]

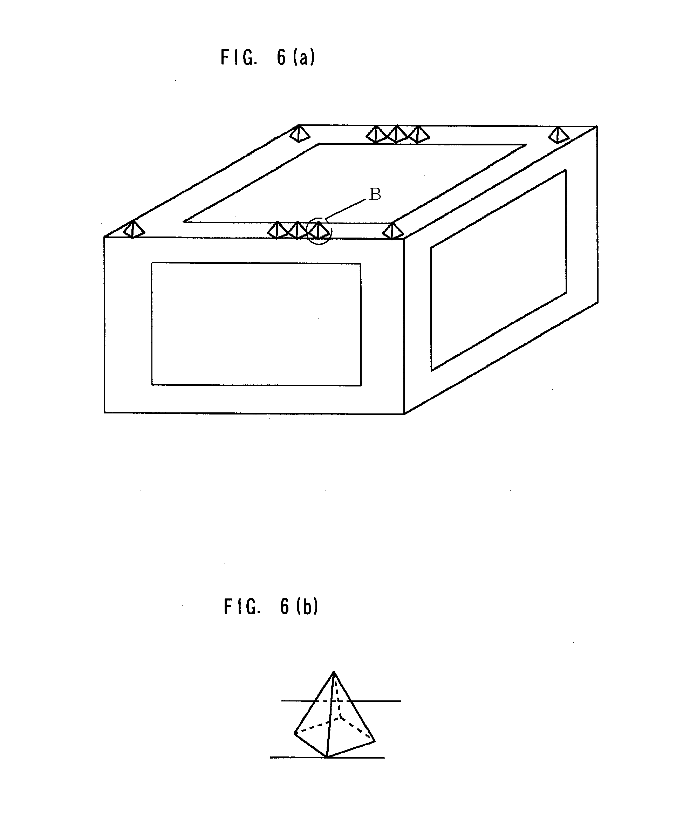

The mixture for dense part formulated in Example 1 was injection-molded at 165°C to obtain a shaped body (a3) having an outer dimension of 30 mm × 20 mm × 15 mm in which a plurality of right quadrangular pyramids of 1.5 mm each on a side were arranged to stand up on one end face of a rectangular parallelepiped frame part made of right quadrangular prisms of 3 mm wide each as shown in Fig. 6.

The shaped body (a3) was placed in a die having an inner dimension that is slightly larger than the outer dimension of the shaped body (a3), and the remaining space was charged with the mixture for porous part formulated in Example 1, and a pressure of 85 MPa was applied to give a combined body in which a shaped body (b3) of an approximately rectangular parallelepiped was molded inside the shaped body (a3).

This combined body was calcined in the same condition as in Example 1. The density of the sintered body corresponding to the dense part was 97%, and the porosity of the sintered body corresponding to the porous part was 61%. The compressive strength was 73 MPa, and the pore diameter of the porous part was within the range of 200 to 500 µm.

The obtained artificial bone was subjected to an alkali and heat treatment, and dipped in a pseudo body fluid in a similar manner as in Example 1, and deposition of an apatite phase was observed in the dense part and in the porous part after three days.

### [Reference Example ]

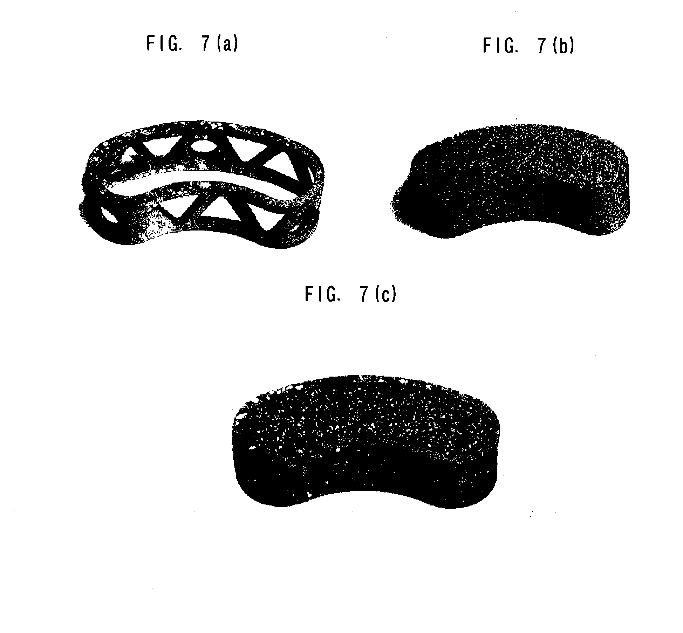

A shaped body (a4) having a soybean-like shape in a planar view as seen in a picture of Fig. 7(a), formed with a plurality of large pores on a lateral face was obtained by processing a pure titanium expanded material of Japanese Industrial Standard II (corresponding to US standard ASTM-G1).

Separately, a mixture for porous part was prepared in the same condition as in Example 1 and placed in a die, and a pressure of 90 MPa was applied, and heated for two hours in argon gas at 900°C to give a presintered body (B4) having an outer shape which is complementary with an inner circumferential face of the shaped body (a4) except for the height higher by 0.5 mm than the shaped body (a4) as shown in a picture of Fig. 7(b).

After fitting the shaped body (a4) with the presintered body (B4), and applying a pressure of 47 MPa in the height direction, heating was conducted in argon gas at 1150°C for two hours to produce an artificial bone as shown in Fig. 7(c).

The porosity of the porous part of the obtained artificial bone was 60%. Also it was found that the dense part and the porous part bind microstructually.

## Claims

1. A method of producing an artificial bone **comprising a dense part having a density of 95% or more, and a porous part having a porosity of 40% or more**, the method comprising the steps of:
- preparing a die having an inner face that is complementary with a living bone;
- placing a shaped body (a) of titanium or a titanium alloy powder not containing a pore forming agent, **the shaped body (a)** having a frame shape that is approximate with a part of an outer face of the living bone and has an opening, in the die;
- charging the remaining space of the die with a mixture containing titanium or a titanium alloy powder and a pore forming agent;
- pressure-molding the mixture, to obtain a shaped body (b) and combine the shaped body (b) with the shaped body (a); and
- calcining the combined body,
**wherein the dense part is derived from the shaped body (a) and the porous part is derived from the shaped body (b).**

2. The method according to claim 1, wherein the part of the outer face of the living bone is a part including a corner, an edge line or a surface of the living bone.

3. The method according to claim 2, wherein the shaped body (a) has a sharp projection on one end face.

4. The method according to any of claims 1 to 3, wherein either one or both of the shaped body (a) and the shaped body (b) contain an organic binder.

5. The method according to claim 4, wherein the organic binder is composed of 0 to 50 vol% of polyacetal, 0 to 50 vol% of polypropylene and 50 to 70 vol% of waxes.

6. The method according to any of claims 1 to 5, wherein the pressure molding is conducted at a pressure of 20 to 100 MPa.

## Patentansprüche

1. Verfahren zum Herstellen eines künstlichen Knochens, der einen dichten Teil mit einer Dichte von 95 % oder mehr und einen porösen Teil mit einer Porosität von 40 % oder mehr umfasst, wobei das Verfahren die Schritte umfasst:
- Herstellen einer Form, die eine Innenfläche aufweist, die komplementär zu einem lebenden Knochen ist,
- Platzieren eines geformten Körpers (a) aus Titan oder einem Titanlegierungspulver, das kein Porenbildungsmittel enthält, wobei der geformte Körper (a) eine Rahmenform aufweist, die einem Teil einer Außenfläche des lebenden Knochens angenähert ist und eine Öffnung aufweist, in der Form,
- Auffüllen des verbleibenden Raums der Form mit einer Mischung, die Titan oder ein Titanlegierungspulver und ein Porenbildungsmittel enthält,
- Druckformen der Mischung, um einen geformten Körper (b) zu erhalten, und Kombinieren des geformten Körpers (b) mit dem geformten Körper (a) und
- Kalzinieren des kombinierten Körpers,
wobei der dichte Teil aus dem geformten Körper (a) erhalten wird und der poröse Teil aus dem geformten Körper (b) erhalten wird.

2. Verfahren nach Anspruch 1, wobei der Teil der Außenfläche des lebenden Knochens ein Teil ist, der eine Ecke, eine Kantenlinie oder eine Oberfläche des lebenden Knochens umfasst.

3. Verfahren nach Anspruch 2, wobei der geformte Körper (a) einen scharfen Vorsprung an einer Endfläche aufweist.

4. Verfahren nach einem der Ansprüche 1 bis 3, wobei entweder einer oder beide von dem geformten Körper (a) und dem geformten Körper (b) ein organisches Bindemittel enthält/enthalten.

5. Verfahren nach Anspruch 4, wobei das organische Bindemittel aus 0 bis 50 Vol-% Polyacetal, 0 bis 50 Vol-% Polypropylen und 50 bis 70 Vol-% Wachsen zusammengesetzt ist.

6. Verfahren nach einem der Ansprüche 1 bis 5, wobei das Druckformen bei einem Druck von 20 bis 100 MPa durchgeführt wird.

## Revendications

1. Procédé de production d'un os artificiel comprenant une partie dense ayant une densité de 95 % ou supérieure et une partie poreuse ayant une porosité de 40 % ou supérieure, le procédé comprenant les étapes consistant :
à préparer une matrice présentant une face interne qui est complémentaire avec un os vivant ;
- à placer un corps façonné (a) de titane ou d'une poudre d'alliage de titane ne contenant pas d'agent formant des pores, le corps façonné (a) présentant une forme de cadre qui est approximative à une partie d'une face externe de l'os vivant et présente une ouverture, dans la matrice ;
- à charger l'espace restant de la matrice avec un mélange contenant du titane ou une poudre d'alliage de titane et un agent formant des pores ;
- à mouler sous pression le mélange, pour obtenir un corps façonné (b) et combiner le corps façonné (b) avec le corps façonné (a) ; et
- à calciner le corps combiné,
dans lequel la partie dense est dérivée du corps façonné (a) et la partie poreuse est dérivée du corps façonné (b).

2. Procédé selon la revendication 1, dans lequel la partie de la face externe de l'os vivant est une partie comprenant un coin, une ligne de bord ou une surface de l'os vivant.

3. Procédé selon la revendication 2, dans lequel le corps façonné (a) présente une saillie pointue sur une face d'extrémité.

4. Procédé selon l'une quelconque des revendications 1 à 3, dans lequel soit l'un, soit les deux du corps façonné (a) et du corps façonné (b) contient un liant organique.

5. Procédé selon la revendication 4, dans lequel le liant organique est constitué de 0 à 50 % en volume de polyacétal, de 0 à 50 % en volume de polypropylène et de 50 à 70 % en volume de cires.

6. Procédé selon l'une quelconque des revendications 1 à 5, dans lequel le moulage sous pression est réalisé à une pression de 20 à 100 MPa.
